Europäisches Patentamt

European Patent Office

Office européen des brevets

(11) Publication number: **0 090 892**
**B1**

# EUROPEAN PATENT SPECIFICATION

(45) Date of publication of patent specification: **12.11.86**

(21) Application number: **82301853.6**

(22) Date of filing: **07.04.82**

(51) Int. Cl.⁴: **C 07 K 3/02, C 07 K 15/06, A 61 K 37/02**

(54) **Process for the purification of physiologically active substance having antitumour activity.**

(43) Date of publication of application:
**12.10.83 Bulletin 83/41**

(45) Publication of the grant of the patent:
**12.11.86 Bulletin 86/46**

(84) Designated Contracting States:
**AT DE GB NL SE**

(56) References cited:

THE JOURNAL OF IMMUNOLOGY, vol. 125, no. 4, October 1980, pages 1671-1677; M.R. RUFF et al.: "Purification and physico-chemical characterization of rabbit tumor necrosis factor".

CHEMICAL ABSTRACTS, vol. 94, no. 11, 16th March 1981, page 257, no. 78873x, Columbus Ohio (USA); N. MATTHEWS et al.: "Tumor-necrosis factor from the rabbit. IV. Purification and chemical characterization".

(73) Proprietor: **Asahi Kasei Kogyo Kabushiki Kaisha**
**2-6, Dojimahama 1-chome Kita-ku**
**Osaka-shi Osaka 530 (JP)**

(73) Proprietor: **Dainippon Pharmaceutical Co., Ltd.**
**25, Doshomachi 3-chome Higashi-ku**
**Osaka-shi, Osaka 541 (JP)**

(72) Inventor: **Haranaka, Katsuyuki**
**Hiyoshi-cho, 2-31-10**
**Kokubunki-shi Tokyo-to (JP)**
Inventor: **Old, Lloyd J.**
**600 West End Avenue**
**New York New York 10021 (US)**
Inventor: **Richards, Elizabeth C.**
**10 Otter Trail**
**Westport Connecticut 06880 (US)**
Inventor: **Williamson, Barbara**
**36 Shore Road**
**Old Greenwich Connecticut 06870 (US)**

(74) Representative: **Myerscough, Philip Boyd et al**
**J.A.Kemp & Co. 14, South Square Gray's Inn**
**London, WC1R 5EU (GB)**

Courier Press, Leamington Spa, England.

## Description

This invention relates to a process for purifying a proteinaceous physiologically active substance having antitumor activity.

There have been a number of reports on the existence of substances having physiological activities such as tumor cell-killing ability, the typical examples of which are briefly reviewed in the following:

Currie et al. discovered that a factor which inhibits the proliferation of various tumor cells was induced by administration of endotoxin to peritoneal exudate cells from a normal rat [J. Exp. Med., Vol. 142 pp. 1600—1605 (1975)], and thereafter further investigation was done on said factor to find that the principal of the factor is arginase as reported in Nature (London), Vol. 273, pp. 758—759 (1978).

Reed et al. also discovered a proteinaceous substance with molecular weight of 45,000 having an ability to kill cultured tumor cells such as L cells from cultured cells or cultured mononuclear supernatant of normal rat and human by applying endotoxin treatment, but the principal of said substance has not yet been isolated and identified [J. Immunol., Vol. 115, pp. 395—404 (1975)].

Carswell et al. discovered that the serum from CD-1 Swiss mouse infected with bacillus Calmette-Guérin (BCG), and after two weeks, followed by intravenous injection of endotoxin has cytotoxic activity against cultured L cells and also a phenomen that it induces hemorrhagic necrosis of transplanted BALB/c sarcoma Meth A in the

(BALB/c×C57BL/6)F₁

mouse, and they gave the name of TNF (Tumor Necrosis Factor) to the active substance in the serum [Proc. Nat. Acad. Sci. USA, Vol. 72 (No. 9), pp. 3666—3670 (1975)]. Further, they conducted partial purification of TNF from said serum and consequently obtained TNF fractions purified 20- to 30-fold over the serum, reporting that the active substance is a glycoprotein having a molecular weight of about 150,000 which migrates with α-globulins in cellulose acetate electrophoresis [Proc. Nat. Acad. Sci. USA, Vol. 73 (No. 2), pp. 381—385 (1976)].

Männel et al. examined the properties of Cytotoxic Factor by the use of the mouse serum obtained by the method of Carswell et al. as mentioned above to find that on the gel filtration the eluted fractions with cytotoxic activity were varied depending on the salt concentration in the buffer and that the molecular weight of Cytotoxic Factor was 55,000 to 60,000 in a buffer with a high salt concentration, while it was 125,000 to 150,000 through aggregation in a buffer with a low salt concentration or serum [Infect. Immun., Vol. 28 (No. 1), pp. 204—211 (1980)]. They reported that the isoelectric point (pI value) of this factor was 4.8. But, the activity of this factor was not evaluated in an animal (for example, evaluation using transplanted Meth A sarcoma in mouse) and therefore the presence of its activity in vivo cannot be ascertained. It is consequently impossible to judge whether the factor is identical with TNF of Carswell et al. Further, they mentioned almost nothing about its purification and isolation, although some properties of it were examined based on cytotoxic activity against L cells.

Matthews et al. induced TNF in a rabbit, examined the properties of the rabbit TNF from the serum and reported that the rabbit TNF had a molecular weight in the range of from 40,000 to 50,000 as measured by gel filtration using Sephadex G-200 [Br. J. Cancer, Vol. 38, pp. 302—309 (1978)]. However, they also reported that the molecular weight was 67,000 on gradient polyacrylamide gel electrophoresis or 39,000 on gel filtration with Ultrogel AcA 44 [Br. J. Cancer, Vol. 42, pp. 416—422 (1980)]. But, since they used no isolated and purified sample, it is not certain whether the TNF is a single substance or not.

Ruff et al. reported that the rabbit TNF was purified 2,000-fold over the serum by a series of operations including salt precipitation using ammonium sulfate, gel filtration, ion exchange chromatography and lectin affinity chromatography. The molecular weight of the rabbit TNF was estimated by them to be 68,000 by SDS-polyacrylamide gel electrophoresis, 55,000 by gel filtration using Sephacryl-200 and 52,000 by glycerol gradient centrifugation [J. Immunol., Vol. 125 (No. 4), pp. 1671—1677 (1980)].

Kull et al. obtained three kinds of tumor cell cytotoxin fractions from the mouse serum obtained by the method of Carswell et al. as mentioned above and conducted tests using transplanted Meth A sarcoma in mouse, reporting that the fraction with molecular weight of 160,000 induced tumor necrosis, while the fractions with molecular weights of 225,000 and 50,000 induced no tumor necrosis [J. Immunol., Vol. 126 (No. 4), pp. 1279—1283 (1981)].

As described above, in spite of a number of reports on the existence of various physiologically active factors, they are not obtained in most cases in amounts sufficient for extensive examination of their properties. In addition, since no isolation is accomplished, it is not certain under the present situation whether they are known substances or novel substances.

Using various kinds of mammals, we have investigated the productivity and purification of a physiologically active substance having antitumor activity which is induced by administering to a mammal a substance having a capacity for stimulating the reticuloendothelial system and injecting endotoxin into the mammal, and found that a rabbit is most suitable for the practical application of the physiologically active substance as a medicine.

The present invention provides a process for purifying a proteinaceous physiologically active substance having antitumor activity, the formation of which physiologically active substance is induced by administering to a rabbit at least one substance having a capacity for stimulating the reticuloendothelial system and then injecting endotoxin from a Gram-negative bacterium into the rabbit, which process comprises the sequential steps of (1) contacting a crude solution of said physiologically active substance which has not been subjected to ammonium sulfate fractionation with a basic anion exchanger using a buffer solution of pH 6.0 to 9.0 and salt concentration of not more than 0.2 M to have said physiologically active substance adsorbed on the anion exchanger, (2) eluting the adsorbed physiologically active substance from the anion exchanger with a buffer solution of higher salt concentration and then (3) after concentrating and optionally dialysing the eluate containing said physiologically active substance, subjecting it to gel filtration with a gel suitable for separation of a substance with a molecular weight, of 30,000 to 70,000 and as an eluent a buffer solution of pH 6.0 to 9.0 to provide said purified proteinaceous physiologically active substance.

In order to induce the physiologically active substance having antitumor activity (hereinafter referred to as the physiologically active substance), at least one substance having a capacity for stimulating the reticuloendothelial system is first injected intravenously or intraperitoneally into a rabbit according to the method of Carswell et al. [Proc. Nat. Acad. Sci. USA, Vol. 72 (No. 9), pp. 3666—3670 (1975)]. As substances having a capacity or stimulating the reticuloendothelial system, there are generally used Gram-positive bacteria, protozoas or yeasts, which are administered to the rabbit in the form of either living microorganisms, dead microorganisms (e.g. after heat treatment or formalin treatment) or microorganism cell extracts. Examples of the Gram-positive bacteria include Propionibacteria such as Propionibacterium acnes (Corynebacterium parvum) or Propionibacterium granulosum (Corynebacterium granulosum), Mycobacteria such as bacillus Calmette-Guérin (BCG) or Mycobacterium smegmatis, and Nocardias such as Nocardia erythropolis or Nocardia gardneri. As a protoza, for example, Plasmodium or Toxoplasma is employable. As yeast, Zymosan extracted from Saccharomyces cerevisiae or others is generally used. There may also be employable synthetic high molecular compounds such as pyran copolymer. Seven to 14 days after administration, endotoxin from a Gram-negative bacterium, for example, a lipopolysaccharide derived from Escherichi coli, Pseudomonas aeruginosa, or Salmonella typhosa is injected intravenously into said rabbit. Then, 1.5 to 2 hours after the injection, body fluids (e.g. ascites, lymph, etc.) and/or serum or plasma of said rabbit are taken or internal organs such as liver, spleen, etc. are homogenized and extracted with physiological saline solution. These body fluids, serum, plasma and/or extract of internal organs are employed as crude solutions of the present physiologically active substance, but it is preferred to employ serum or plasma.

Evaluation of the physiological activity of the physiologically active substance is conducted according to the following methods.

a) Evaluation using L cell

This is conducted according to the method of Carswell et al. [Proc. Nat. Acad. Sci. USA, Vol. 72 (No. 9), pp. 3666—3670 (1975)]. As a culture vessel, there is employed a plate produced by Lymbro Chemical Co., Inc. (U.S.A.) and L cells (S) are cultured in Eagle's minimum essential medium (MEM medium) containing non-essential amino acids and 10% heat-inactivated fetal calf serum, further 100 units/ml of penicillin and 100 µg/ml of streptomycin. Equal volumes of an L cell suspension ($1 \times 10^5$ cells) and a serially diluted sample are mixed and incubated at 37°C for 48 hours in an air containing 5% carbon dioxide. The activity is determined by plotting the dilution versus the number of viable L cells on a graph and calculating from the dilution corresponding to 50% cytotoxicity, the ability to kill 50% of L cells. The physiological activity necessary for killing 50% of L cells is defined as 1 unit.

b) Evaluation using transplanted Meth A sarcoma in mouse

According to the method of Carswell et al. (the same literature as cited above), $2 \times 10^5$ BALB/c sarcoma Meth A cells are transplanted intradermally at armpit of a

$$(BALB/c \times C57BL/6)F_1$$

mouse, and 7 days later, mice with tumors of 7—8 mm in diameter, good vascularization and no spontaneous central necrosis are selected for evaluation. A sample (0.5 ml) diluted with physiological saline solution is injected through the tail vein. The activity of the sample is evaluated after 25 hours according to the following criterion.

(−): no change
(+): slight hemorrhagic necrosis
(++): moderate hemorrhagic necrosis (central necrosis extending over approximately 50% of the tumor surface)
(+++): marked hemorrhagic necrosis (massive necrosis leaving a small viable rim along the tumor periphery)

In the following, the process for purification of this invention is to be described in detail.

Purification step A

Prior to the first step of contacting with a basic anion exchanger, a crude solution of the physiologically active substance may be dialyzed against a buffer solution to be used at the time of contacting with an anion exchanger or it may be diluted with a buffer solution having a low salt concentration.

The contact of a crude solution of the physiologically active substance with a basic anion exchanger can be conducted by either column method or batch method. This step is carried out by contacting the crude solution with a basic anion exchanger using a buffer solution of pH 6.0 to 9.0 and a salt concentration of 0.2 M or lower to have the physiologically active substance adsorbed on the anion exchanger, subsequently washing said anion exchanger with the same buffer solution to remove the unadsorbed proteins and thereafter eluting the physiologically active substance using a buffer solution of a higher salt concentration.

Typical examples of basic anion exchangers used include anion exchangers containing diethylamino ethyl groups such as DEAE-Sephadex® A-50, DEAE-Sepharose® CL-6B, DEAE-Sephacel® (all produced by Pharmacia Fine Chemicals AB, Sweden) and AIEC®DE 52 (produced by Whatman Ltd., England), anion exchangers containing aminoethyl groups such as Servacel® AE (produced by Serva Entwicklungslabor, West Germany), and anion exchangers containing quaternized aminoethyl groups such as QAE-Sephadex® A-50 (produced by Pharmacia) and Cellex® QAE (produced by Bio-Rad Laboratories, U.S.A.). The buffer solutions used include a dilute Tris-hydrochloric acid buffer and a dilute phosphate buffer. Sodium chloride or potassium chloride is preferably added to adjust a salt concentration of the buffer solution. The content of protein in an eluate is determined by the optical density at 280 nm. The concentration of the present physiologically active substance is measured as the cytotoxic activity against L cells as described above.

Although the above step can be carried out with a single contact with the anion exchanger, it is sometimes preferable in the case of column method to employ re-chromatography.

Purification step B

The eluate containing the physiologically active substance obtained in the preceding step is concentrated by a conventional method such as ultrafiltration or lyophilization. The thus obtained concentrate is subjected to gel filtration using a gel suitable for separation of a substance with a molecular weight of 30,000 to 70,000. As an eluent, there is employed a buffer solution having a pH generally of 6.0 to 9.0. The salt concentration is not critical, but preferably is 0.15 to 2.0 M. The gels for gel filtration include Sephadex® G-75, 100, 150 or 200 (produced by Pharmacia), Sephacryl® S-200 or 300 (produced by Pharmacia), Bio-Gel® P-30, 60, 100, 150 or 200 (produced by Bio-Rad), and CPG-10 (350 Å, 240 Å, 170 Å, or 120 Å) (produced by Electro-Nucleonics, Inc., U.S.A.). Examples of the buffer solution and the salt are the same as those described above in the step of the contact with an anion exchanger.

The fractions containing the physiologically active substance are pooled and concentrated by a conventional method such as ultrafiltration or lyophilization. The dialysis of the concentrate of the present physiologically active substance against a physiological saline solution affords a solution of said substance purified 5,000- to 10,000-fold over the serum or plasma. The overall activity recovery of the two steps according to the evaluation using L cell is 65 to 98%.

The thus prepared purified solution of the physiologically active substance is adjusted to appropriate pH and salt concentration by dialysis or gel filtration, sterilized by filtration, and if necessary, heated, and lyophilized to give purified preparation of the physiologically active substance.

The purified preparation in an amount of about 3,000 units was found to exhibit (++) activity in the evaluation using Meth A sarcoma as described above. The purified preparation of the present physiologically active substance was also found to exhibit cytotoxic activity against various cultured human cancer cell lines. The percent cytotoxicity at 48 hours after administration of 800 units of the purified preparation is shown in Table 1.

TABLE 1

| Cancer cell lines | Percent cytotoxicity | Medium* |
|---|---|---|
| PC 10 | 69.1 | a |
| KATO-III | 67.9 | b |
| MK-7 | 65.8 | a |
| Rca | 66.8 | c |
| W-2 | 75.9 | a |
| GOTO | 61.3 | b |
| SEKI | 70.0 | b |
| Kym-1 | 51.9 | d |
| MRK-1-nu | 75.1 | d |

* a: 80% RPMI 1640+20% FCS
  b: 40% RPMI 1640+40% MEM+20% FCS
  c: 80% MEM+20% FCS
  d: 80% DM160+20% FCS

On the other hand, the purified preparation of the physiologically active substance was found to exhibit no cytotoxic activity against normal cells such as cultured fibroblasts of human and mouse even in a dose of $2 \times 10^6$ units.

Further, in the tests in which the purified preparation of the physiologically active substance was administered to a BALB/c mouse with transplanted Colon 26 adenocarcinoma and a A/Jax mouse with transplanted Neuro-2a neuroblastoma, there were seen significant growth inhibition and regression of the tumors as compared with Control Group (Group to which physiological saline solution was administered). The grown tumors regressed or disappeared without causing hemorrhagic necrosis in some animals.

The purified preparation of the physiologically active substance has an excellent antitumor activity, which is less in species specificity, and it can be used as an antitumor agent for the treatment of malignant tumors in a mammal including human.

The purified preparation of the physiologically active substance is generally administered parenterally or topically in the form of an aqueous solution to which an isotonic agent such as sodium chloride or/and a buffering agent such as phosphate may be optionally added. The clinical dosage of the purified preparation of the physiologically active substance, which may vary depending on the route of administration, the conditions as well as the body weight of the patient, is generally $10^4$ to $10^8$ units per one administration for a human adult. The purified preparation of the physiologically active substance may also be used in combination with other antitumor agents such as cyclophosphamide, mitomycin C, adriamycin and bleomycin.

The purified preparation of the physiologically active substance obtained as described above can further be subjected to the steps as shown below, whereby the physiologically active substance can be isolated:

(C) Affinity chromatography on immobilized Cibacron® Blue F3G-A;

(D) Gel filtration;

(E) Affinity chromatography on immobilized concanavalin A;

(F) Preparative electrophoresis on polyacrylamide gel; and

(G) Gel filtration.

Each of these steps is to be described in detail below.

Purification step C

The concentrate of the physiologically active substance from step B is subjected to affinity chromatography on immobilized Cibacron® Blue F3G-A (dye produced by Ciba-Geigy Corp.). Immobilization of Cibacron Blue F3G-A on a support may be performed according to a known method as described by Böhme et al. in J. Chromatogr., Vol. 69, pp. 209—214 (1972), or alternatively commercially available adsorbent [e.g. Blue Sepharose® CL-6B (produced by Pharmacia), Affi-Gel Blue ® (produced by Bio-Rad)] may be used. After the concentrate of the physiologically active substance is dialyzed against a dilute buffer solution of pH 7.0 to 8.0 (e.g. phosphate buffer or Tris-hydrochloric acid buffer), it is applied to the above immobilized Cibacron Blue F3G-A. By this operation, contaminating proteins such as albumin are adsorbed on immobilized Cibacron Blue F3G-A and the physiologically active substance is eluted in unadsorbed fractions. The activity recovery at this step is about 70 to 95% with about 3-fold increase in purity. The overall activity recovery through the purification steps A to C is about 56 to 93% with the purity being about $1.5 \times 10^4$- to $3 \times 10^4$-fold.

Purification step D

The unadsorbed fractions from step C are concentrated and the concentrate is subjected to gel filtration under the same conditions as for step B. As the support for gel filtration, there may be employed Sephadex® G-75, 100, 150 or 200 (produced by Pharmacia), Bio-Gel® P-30, 60, 100, 150 or 200 (produced by Bio-Rad). The active fractions are pooled, concentrated and dialyzed against a dilute phosphate buffer or Tris-hydrochloric acid buffer of pH 7.0 to 8.0. The activity recovery at this step is about 70 to 95% with about 3- to 4-fold increase in purity.

The overall activity recovery through the purification steps A to D is about 47 to 88% with the purity being about $4.5 \times 10^4$- to $9 \times 10^4$-fold.

Purification step E

The purified solution from step D is subsequently subjected to affinity chromatography using immobilized concanavalin A.

Concanavalin A can be immobilized by a known method or alternatively a commercially available immobilized concanavalin A (produced by Sigma Chemical Co., U.S.A.) or Con A-Sepharose® CL-6B (produced by Pharmacia) may be used. The purified solution from step D is concentrated and applied to immobilized concanavalin A using a dilute buffer solution of pH 7.0 to 8.0 as used in the same step, and then, after washing thoroughly the column with the same buffer solution, elution is performed with the same buffer solution containing 0.1 M or more of α-methyl-d-mannoside. The physiologically active substance is concentrated in unadsorbed fractions. The activity recovery at this step is about 60 to 80% with about 2- to 4-fold increase in purity. The overall activity recovery through the purification steps A to E is about 33 to 70% with the purity being about $9.0 \times 10^4$- to $3.6 \times 10^5$-fold.

Purification step F

The solution containing the physiologically active substance from step E is concentrated and subjected to polyacrylamide-slab electrophoresis. The concentrate of the physiologically active substance is applied on an 8% polyacrylamide gel prepared by the use of slab electrophoresis apparatus Model 221 ($280 \times 140 \times 1.5$ mm) produced by Bio-Rad Laboratories. Electrophoresis is performed while maintaining the current at about 70 to 100 mA. After migration, the gel is cut into strips each of 3 mm width and each gel strip is extracted with a dilute buffer solution of pH 7.0 to 8.0 containing 1.0 M sodium chloride, and the active fractions are pooled and concentrated. The activity recovery at this step is about 5 to 10% with about 15- to 20-fold increase in purity. The overall activity recovery through the purification steps A to F is about 2.5 to 7.0% with the purity being about $1.4 \times 10^6$- to $5.4 \times 10^6$-fold.

Purification step G

On gel filtration of this step, the same buffer, salt and gel as employed in step B can be used. But the length and the diameter of the column to be used in this step are longer and smaller, respectively, than those of the column employed in step B. The active fractions are pooled, concentrated, dialyzed, sterilized by filtration and, if necessary, lyophilized to provide the novel physiologically active substance of the present invention. The activity recovery at this step is about 50 to 80% with about 1.5- to 2.0-fold increase in purity. The overall activity recovery through the purification steps A to G is 1.6 to 5.6% with the purity being $2.8 \times 10^6$- to $8.1 \times 10^6$-fold.

The characteristics of the physiologically active substance thus prepared were measured to obtain the results shown below:

a) Molecular weight
   $39,000 \pm 5,000$ (by SDS-polyacrylamide gel electrophoresis and gel filtration)

b) Isoelectric point
   pH $3.9 \pm 0.3$

c) Cellulose acetate electrophoresis mobility
   $10^{-4}$ to $10^{-6}$ cm²/v · sec

d) Specific activity by evaluation using L cell
   at least $0.5 \times 10^9$ units/mg-protein
   Further, $2 \times 10^5$ Meth A sarcoma cells were transplanted intradermally at the armpit of

$(BALB/c \times C57BL/6)F_1$

mouse and permitted to proliferate sufficiently to form solid tumors and thereafter the physiologically active substance was administered intravenously (at a dose corresponding to 0.1 to 1 ng of protein per mouse), whereby activities of (+) or higher were exhibited.

Among the above characteristics, a) to c) were measured according to the following methods:

a) Determination of molecular weight
   (i) According to the method of Segrest et al · [Methods in Enzymology Vol. 28-B, pp. 54—63 (1972)] 5 μg of a sample is applied on SDS (sodium dodecyl sulfate)-polyacrylamide gel and electrophoresis is carried out in SDS/Tris-glycine buffer (pH 8.3). Calibration of the molecular weight is conducted by the use of a standard molecular weight kit (produced by Pharmacia).
   (ii) Using a column (0.9×120 cm) of Sephadex G-200 (produced by Pharmacia), gel filtration is performed with buffer solution of 0.7 M sodium chloride/0.02 M Tris-hydrochloric acid buffer (pH 7.8), and calibration of the molecular weight is conducted by the use of standard proteins (ribonuclease A, chymotrypsinogen A, ovalbumin, aldonase, produced by Pharmacia).

b) Determination of isoelectric point
   The isoelectric point is determined using the apparatus for isoelectric electrophoresis, Ampholine (pH range 2.5 to 4.5) and 36% Ultrodex (all produced by LKB Productor AB, Sweden). The formation of pH gradient is effected at 340 V and 23 mA for 5 to 7 hours and thereafter sample is applied on the slab. Migration is conducted up to 660 V and 120 mA for 5 to 7 hours. Strips with width of 1 mm are prepared and extracted with physiological saline solution and cytotoxic activity against L cells is measured.

c) mobility in electrophoresis
   Using Separax-S (produced by Fuji Photo Film Co., Ltd., Japan) as cellulose acetate membrane, electrophoresis is performed at pH 8.6 and ionic strength of 0.06 to 0.07. After completion of migration, strips with width of 1 mm are prepared, extracted with physiological saline solution and evaluated for cytotoxic activity against L cells to determine the mobility.

The physiologically active substance was tested for its cytotoxic activity against various cultured human cancer cell lines. Table 2 shows the results in terms of the corresponding protein amount necessary for 50% cytotoxicity after 48 hours.

TABLE 2

| Cancer cell lines | Amount necessary for 50% cytotoxicity (pg) | Medium* |
|---|---|---|
| PC 10 | $5 \times 10^2$ | a |
| KATO-III | $9 \times 10^2$ | b |
| MK-7 | $9 \times 10^2$ | a |
| Rca | $6 \times 10^2$ | c |
| W-2 | $8 \times 10^2$ | a |
| GOTO | $1 \times 10^3$ | b |
| SEKI | $3 \times 10^2$ | b |
| Kym-1 | $1 \times 10^3$ | d |
| MRK-1-nu | $7 \times 10^2$ | d |

* a: 80% RPMI 1640+20% FCS
  b: 40% RPMI 1640+40% MEM+20% FCS
  c: 80% MEM+20% FCS
  d: 80% DM160+20% FCS

The present invention is further illustrated in detail by referring to the following Examples, by which the present invention is not limited.

Example 1

Female rabbits, weighing 2.5 to 3 kg, were injected with 50 mg of formalin-killed Propionibacterium acnes (Corynebacterium parvum; Wellcome Research Laboratories, England) through the ear vein. Eight days later, 100 µg of endotoxin (lipopolysaccharide from Escherichia coli 026:B6, produced by Difco Laboratories, U.S.A.) was injected again through the ear vein and 2 hours later whole blood was collected from the heart. The collected blood was centrifuged at 5,000 rpm for 30 minutes to remove blood cells and insoluble solids. From 20 rabbits, 1,200 ml of serum having an activity of 12,800 units/ml was obtained.

The serum was diluted with 600 ml of 0.02 M Tris-hydrochloric acid buffer (pH 7.8) and applied slowly to a column (6×36 cm) of DEAE-Sepharose® CL-6B (Pharmacia) equilibrated with 0.02 M Tris-hydrochloric acid buffer (pH 7.8) containing 0.1 M sodium chloride. Then, after washing the column with 1,000 ml of equilibrating buffer (0.02 M Tris-hydrochloric acid buffer, pH 7.8, containing 0.1 M sodium chloride), elution was carried out with the NaCl-linear gradient formed by a gradienter, using 1.5 liters of 0.02 M Tris-hydrochloric acid buffer (pH 7.8) containing 0.1 M sodium chloride and 1.5 liters of 0.02 M Tris-hydrochloric acid buffer (pH 7.8) containing 0.3 M sodium chloride. The flow rate was 60 ml/hour and fractions each of 18 ml were collected. The active fractions were pooled and concentrated. The activity recovery at this step was 92% with 150-fold increase in purity.

Then, the concentrate was dialyzed against 0.005 M phosphate buffer (pH 7.4), containing 0.15 M sodium chloride overnight and gel-filtered. A column (5×80 cm) of Sephacryl® S-200 (Pharmacia) was sufficiently equilibrated with the same buffer, and the concentrate was applied to the column and elution conducted with the same buffer. The flow rate was 60 ml/hour and fractions each of 10 ml were collected. The active fractions obtained immediately after the albumin fraction were concentrated by ultrafiltration to obtain a purified preparation of the physiologically active substance. At this step, the activity recovery was 92% with 52-fold increase in purity. The overall activity recovery through all the steps was 85% with the purity being 7,800-fold. The purified preparation of the physiologically active substance was found to have a specific activity of about $1.4 \times 10^6$ units/mg-protein.

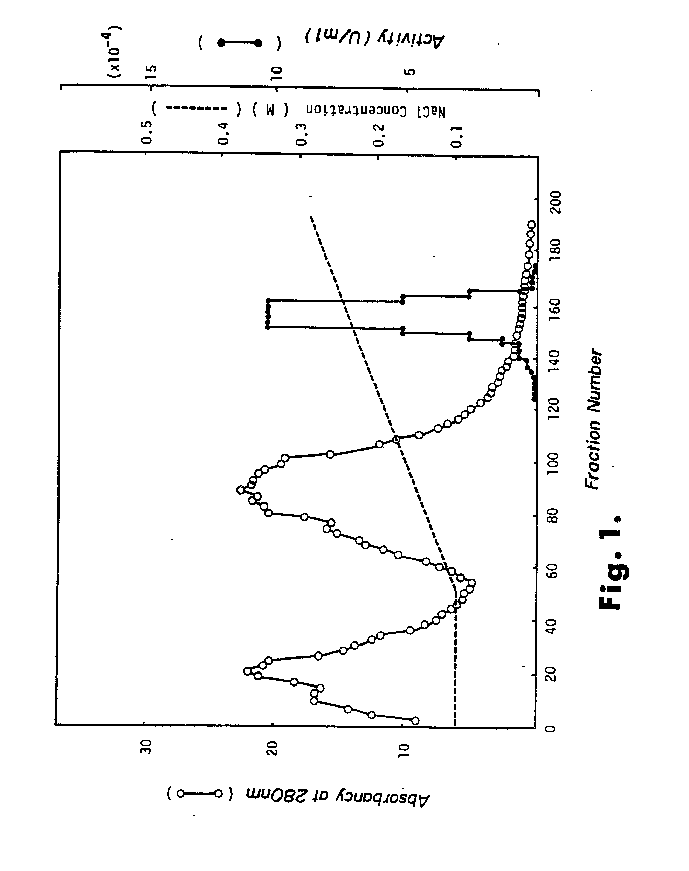

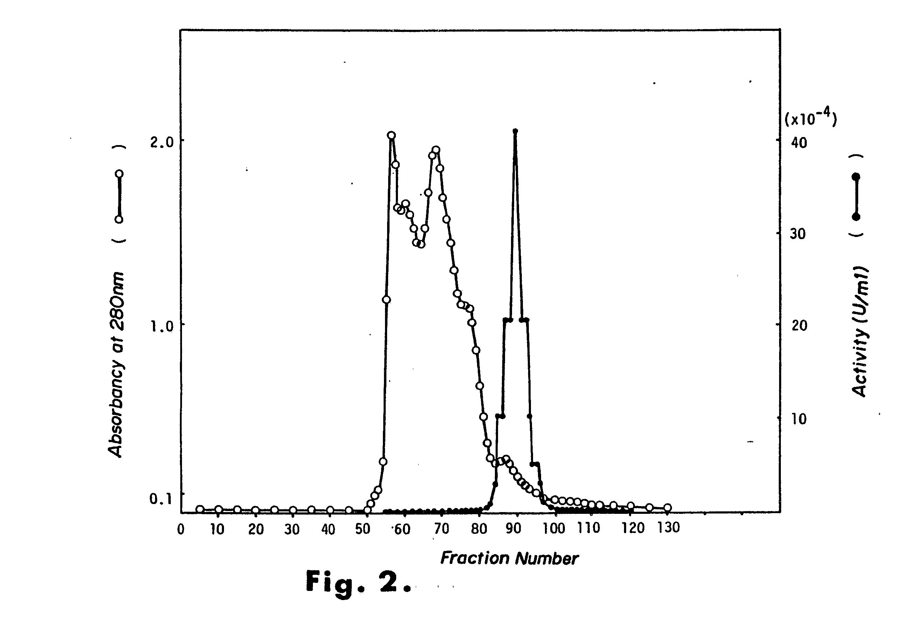

Fig. 1 and Fig. 2 show the patterns of chromatography on DEAE-Sepharose® CL-6B column at the first step and gel filtration on Sephacryl® S-200 column at the second step, respectively.

Example 2

After 2,200 ml of a rabbit serum containing the physiologically active substance was diluted with 1,200 ml of 0.02 M Tris-hydrochloric acid buffer (pH 7.2), the resulting solution was slowly applied to a column (8×26 cm) of DEAE-Sepharose® CL-6B equilibrated with 0.02 M Tris-hydrochloric acid buffer (pH 7.2) containing 0.1 M sodium chloride. Then, the column was washed with 1,000 ml of 0.02 M Tris-hydrochloric acid buffer (pH 7.2) containing 0.13 M sodium chloride, and elution was carried out with the NaCl-linear gradient formed by a gradienter, using 2.0 liters of 0.02 M Tris-hydrochloric acid buffer (pH 7.2) containing 0.15 M sodium chloride and 2.0 liters of 0.02 M Tris-hydrochloric acid buffer (pH 7.2) containing 0.3 M sodium chloride. The flow rate was 90 ml/hour and fractions each of 18 ml were collected.

The active fractions pooled were dialyzed against 0.02 M Tris-hydrochloric acid buffer (pH 7.2) containing 0.1 M sodium chloride, and subjected to rechromatography on a column (2.5×30 cm) of DEAE-Sepharose® CL-6B equilibrated with the same buffer. The physiologically active substance adsorbed was eluted with the NaCl-linear gradient formed by a gradienter, using 350 ml of equilibrating buffer and 350 ml of 0.02 M Tris-hydrochloric acid buffer (pH 7.2) containing 0.3 M sodium chloride. The flow rate was 25 ml/hour and fractions each of 7 ml were collected. The active fractions were pooled and concentrated.

As the next step, the concentrate was applied to a column of Sephacryl® S-200 equilibrated with 0.01 M Tris-hydrochloric acid buffer (pH 7.8) containing 1.0 M sodium chloride and elution was performed with the same buffer. These were collected and lyophilized to obtain a purified preparation of the physiologically active substance having a purity of 10,000-fold higher than the serum. The activity recovery through all the steps was 80%.

Example 3

The purified concentrate obtained in Example 1 was dialyzed against 0.02 M phosphate buffer (pH 7.1) overnight and applied to a column (0.7×10 cm) of Blue Sepharose® CL-6B equilibrated with the same buffer. The column was washed thoroughly with the same buffer and the adsorbed substances were eluted with 50 ml of 0.02 M phosphate buffer (pH 7.1) containing 1.5 M sodium chloride. The flow rate was 2.5 ml/hour and fractions each of 3 ml were collected. No activity was detected in adsorbed fractions, but all activities were recovered in unadsorbed fractions. The active fractions were pooled and lyophilized. At this step, the activity recovery was 95% with 3-fold increase in purity. The overall values through all the steps were 81% for activity recovery and $2.3 \times 10^4$-fold for purity.

As the next step, the lyophilized product was dissolved in 1 ml of 0.02 M Tris-hydrochloric acid buffer (pH 7.8) containing 0.7 M sodium chloride and applied to a column (2.6×95 cm) of Sephadex® G-75 (Pharmacia) equilibrated with the same buffer. The flow rate was 20 ml/hour and fractions each of 10 ml were collected. The active fractions were pooled and concentrated. At this step, the activity recovery was 95% with 3-fold increase in purity. The overall values through all the steps were 77% for activity recovery and $6.9\times10^4$-fold for purity.

The concentrate was subsequently dialyzed against 0.02 M phosphate buffer (pH 7.2) containing 4 M magnesium chloride and applied to a column (0.3×10 cm) of Concanavalin A-Sepharose® CL-6B (Pharmacia), equilibrated with the same buffer, at a flow rate of 2.5 ml/hour. After the column was washed thoroughly with the same buffer, elution was performed with the phosphate buffer containing 0.1 M α-methyl-d-mannoside. The activity was recovered in the unadsorbed fractions and no activity detected in the adsorbed fractions. The unadsorbed fractions were concentrated and dialyzed against the phosphate buffer containing 0.15 M sodium chloride. At this step, the activity recovery was 70% with two-fold increase in purity. The overall activity recovery was 54% with the purity being $1.4\times10^5$-fold.

The dialyzed solution was then applied on 8% polyacrylamide gel prepared by means of Bio-Rad slab electrophoresis apparatus Model 221 (280×140×1.5 mm) and electrophoresis was carried out at a constant current of 80 mA. After electrophoresis, the gel was cut into strips each of 3 mm width, each strip was extracted with 0.05 M Tris-hydrochloric acid buffer (pH 7.8) containing 1.0 M sodium chloride for 24 hours, and the active fractions were pooled and concentrated. At this step, the activity recovery was 8% with 15-fold increase in purity. The overall activity recovery was 4.3% with the purity being $2.1\times10^6$-fold.

Several lots of the physologically active substance purified up to this step were pooled and applied to a slim column (0.9×120 cm) of Sephadex® G-200 equilibrated with 0.05 M Tris-hydrochloric acid buffer (pH 7.8) containing 0.5 M sodium chloride. The flow rate was 3.5 ml/hour and fractions each of 0.3 ml were collected. The active fractions were pooled, concentrated and dialyzed against physiological saline solution to obtain a solution of the physiologically active substance. At this step, the activity recovery was 70% with 1.5-fold increase in purity. The overall activity recovery through all the seven steps was 3.0% with the purity being $3.2\times10^6$-fold. The physiologically active substance was found to have a specific activity of about $0.57\times10^9$ units/mg-protein.

## Claims

1. A process for purifying a proteinaceous physiologically active substance having antitumor activity, the formation of which physiologically active substance is induced by administering to a rabbit at least one substance having a capacity for stimulating the reticuloendothelial system and then injecting endotoxin from a Gram-negative bacterium into the rabbit, which process comprises the sequential steps of (1) contacting a crude solution of said physiologically active substance which has not been subjected to ammonium sulfate fractionation with a basic anion exchanger using a buffer solution of pH 6.0 to 9.0 and salt concentration of not more than 0.2 M to have said physiologically active substance adsorbed on the anion exchanger, (2) eluting the adsorbed physiologically active substance from the anion exchanger with a buffer solution of higher salt concentration and then (3) after concentrating and optionally dialysing the eluate containing said physiologically active substance, subjecting it to gel filtration with a gel suitable for separation of a substance with a molecular weight of 30,000 to 70,000 and as an eluent a buffer solution of pH 6.0 to 9.0 to provide said purified proteinaceous physiologically active substance.

2. A process according to claim 1 wherein the salt concentration of the eluent on gel filtration is 0.15 to 2M.

3. A proteinaceous physiologically active substance, the formation of which is induced by administering to a rabbit at least one substance having a capacity for stimulating the reticuloendothelial system and then injecting endotoxin from a Gram-negative bacterium into the rabbit, having the following characteristic properties:

a) Molecular weight: 39,000±5,000 (determined by SDS-polyacrylamide gel electrophoresis and gel filtration);

b) Isoelectric point; pH 3.9±0.3;

c) Mobility in cellulose acetate electrophoresis (pH 8.6): $10^{-4}$ to $10^{-6}$ cm²/V.sec;

d) Specific activity according to the biological evaluation using L cell as defined in the specification: at least $0.5\times10^9$ units/mg-protein; and

e) Activity according to the biological evaluation using transplanted Meth A sarcoma in

$$(BALB/c\times C57BL/6)F_1$$

mouse as defined in the specification, when administered intravenously at a dose corresponding to 0.1 to 1 ng protein per mouse: (+) or higher.

4. Un aqueous solution of a proteinaceous physiologically active substance purified by a process as claimed in claim 1 or 2, optionally containing an isotonic agent and/or a buffering agent, for use in parenteral or topical administration in the treatment of malignant tumors in mammals including humans.

## Patentansprüche

1. Verfahren zur Reinigung einer physiologisch aktiven Protein-Substanz mit Antitumoraktivität, wobei die Bildung dieser physiologisch aktiven Substanz induziert wird durch Verarbreichung mindestens einer Substanz, die im Stande ist, das retikuloendotheliale System zu stimulieren, an ein Kaninchen und anschließende Injektion von Endotoxin von einem gram-negativen Bakterium in das Kaninchen, umfaßend die aufeinanderfolgenden Stufen von

(1) Zusammenbringen einer rohen Lösung der physiologisch aktiven Substanz, die keiner Amoniumsulfat-Fraktionierung unterworfen worden ist, mit einem basischen Ionenaustauscher unter Verwendung einer Pufferlösung mit pH 6,0 bis 9,0 und einer Salzkonzentration von nicht mehr als 0,2 m, um die physiologisch aktive Substanz an den Anionenaustauscher zu adsorbieren;

(2) Eluieren der adsorbierten physiologisch aktiven Substanz von dem Anionenaustauscher mit einer Pufferlösung von höherer Salzkonzentration und dann

(3) Unterwerfen des diese physiologisch aktive Substanz enthaltenden Eluats- nach Einengen und gegebenenfalls und Dialysieren- der Gelfiltration mit einem Gel, das geeignet ist zur Abtrennung einer Substanz mit einem Molekulargewicht von 30 000 bis 70 000 und unter Verwendung einer Pufferlösung mit pH 6,0 bis 9,0 als Eluens, um die gereinigte physiologisch aktive Proteinsubstanz zu erhalten.

2. Verfahren nach Anspruch 1, wobei die Salzkonzentration des Eluens der Gelfiltration 0,15 bis 2 m ist.

3. Physiologisch aktive Protein-Substanz, deren Bildung induziert wird durch Verabreichung mindestens einer Substanz, die im Stande ist, das retikuloendotheliale System zu stimulieren, an ein Kaninchen und anschließende Injektion von Endotoxin von einem gram-negativen Bakterium in das Kaninchen, und die die folgenden Eigenschaften besitzt:

a) Molekulargewicht: 39 000±5 000 (bestimmt durch SDS - Polyacrylamid - Gelelektrophorese und Gelfiltration)

b) Isoelektrischer Punkt: pH 3,9±0,3;

c) Beweglichkeit bei der Elektrophorese in Cellulloseacetat (pH 8,6): $10^{-4}$ bis $10^{-6}$ cm²/V.s.,

d) spezifische Aktivität entsprechend der biologischen Bewertung unter Verwendung einer L Zelle, wie in der Beschreibung definiert: mindestens $0,5\times10^{-9}$ Einheiten/mg Protein und

e) Aktivität entsprechend der biologischen Bewertung unter Verwendung von transplantiertem Meth A Sarcom in

(BALB/c×C57BL/6)F$_1$

Mäuse, wie in der Beschreibung definiert bei intravenöser Verabreichung in einer Dosis entsprechend 0,1 bis 1 ng Protein pro Maus: (+) oder höher.

4. Wässrige Lösung einer physiologisch aktiven Protein-Substanz stanz, die nach einem Verfahren wie in Anspruch 1 oder 2 angegeben gereinigt worden ist und die gegebenenfalls ein isotonisches Mittel und/oder Puffermittel enthält, zur Verwendung zur patenteralen oder topischen Verarbreichung bei der Behandlung bösartiger Tumoren bei Säugetieren einschließlich Menschen.

**Revendications**

1. Procédé de purification d'une substance protéinée physiologiquement active, ayant une activité anti-tumorale, la formation de cette substance physiologiquement active étant induite par administration à un lapin d'au moins une substance ayant une capacité de stimulation du système réticuloendothélial et par injection ultérieure au lapin d'une endotoxine tirée d'une bactérie Gram-négative, lequel procédé comprend les étapes successives de (1) mise en contact d'une solution brute de ladite substance physiologiquement active, qui n'a pas été soumise à un fractionnement au sulfate d'ammonium, avec un échangeur d'anions basique, en utilisant une solution tampon de pH 6,0 à 9,0, et d'une concentration de sel non supérieure à 0,2 M, pour obtenir ladite substance physiologiquement active adsorbée sur l'échangeur d'anions, (2) l'élution, hors de l'échangeur d'anions, de la substance physiologiquement active adsorbée, à l'aide d'une solution tampon de concentration de sel supérieure et puis, (3) après concentration et dialyse éventuelle de l'éluat contenant ladite substance physiologiquement active, la soumission de celui-ci à une filtration sur gel, à l'aide d'un gel convenable pour la séparation d'une substance ayant une masse moléculaire de 30 000 à 70 000, et, en tant qu'éluant, d'une solution tampon de pH 6,0 à 9,0, pour fournir ladite substance protéinée physiologiquement active purifiée.

2. Procédé conforme à la revendication 1, dans lequel la concentration de sel de l'éluant utilisé pour la filtration sur gel est de 0,15 à 2 M.

3. Substance protéinée physiologiquement active, dont la formation est induite par administration à un lapin d'au moins une substance ayant une capacité de stimulation du système réticuloendothélial et par injection ultérieure au lapin d'une endotoxine tirée d'une bactérie Gram-négative, et qui possède les propriétés caractéristiques suivantes:

a) masse moléculaire: 39 000±5 000 (déterminée par électrophorèse sur gel de SDS-polyacrylamide et par filtration sur gel)

b) point isoélectique: pH 3,9±0,3;

c) mobilité dans l'électrophorèse sur acétate de cellulose (pH 8,6): $10^{-4}$ à $10^{-6}$ cm²/V.s;

d) activité spécifique, selon l'évaluation biologique utilisant de cellules L, telle que définie dans la description: au moins $0,5\times10^{9}$ unités/mg de protéine; et

e) activité, selon l'évaluation biologique utilisant un sarcome Meth A transplanté dans des souris

(BALB/c×C57BL/6)F$_1$,

telle que définie dans la description, quand la substance est administrée par voie intraveineuse à une dose correspondant à 0,1 à 1 ng de protéine par souris: (+) ou supérieur.

4. Solution aqueuse d'une substance protéinée physiologiquement active, purifiée par un procédé conforme à la revendication 1 ou 2, contenant éventuellement un agent isotonique et/ou un agent de tamponnage, pour utilisation en administration parentérale ou topique dans le traitement de tumeurs malignes chez les mammifères, y comprise chez l'homme.

Fig. 1.

**Fig. 2.**

Activity (U/ml) ( ●———● )   ($\times 10^{-4}$)

40
30
20
10

Fraction Number

0  10  20  30  40  50  60  70  80  90  100  110  120  130

2.0
1.0
0.1

Absorbancy at 280nm ( ○———○ )